# EUROPÄISCHE PATENTANMELDUNG

(11) **EP 1 808 174 A1**
(43) Veröffentlichungstag der Anmeldung: **18.07.2007**
(21) Anmeldenummer: 07104722.9
(22) Anmeldetag: 09.11.2001
(51) Int. Cl.: A61K 31/46, A61K 31/137, A61P 11/00, A61P 11/06

(54) **Neue Arzneimittelkompositionen auf der Basis von Tiotropiumsalzen und Salzen des Salmeterols**

(30) Priorität: 13.11.2000 DE 10056104
(62) Teilanmeldung aus: 04028508.2
(71) Anmelder: Boehringer Ingelheim Pharma GmbH & Co. KG, 55216 Ingelheim am Rhein (DE)
(72) Erfinder: SCHMELZER, Christel, 55218, Ingelheim am Rhein (DE); NAGEL, Juergen, 55543, Bad Kreuznach (DE)
(74) Vertreter: Hammann, Heinz

(57) **Zusammenfassung**

Die vorliegende Erfindung betrifft neurtige Arzneimittelkompositionen auf der Basis von Tiotropiumsalzen und Salzen des Salmeterols, Verfahren zu deren Herstellung sowie deren Verwendung bei der Therapie von Atemwegserkrankungen.

## Beschreibung

Die vorliegende Erfindung betrifft neurtige Arzneimittelkompositionen auf der Basis von Tiotropiumsalzen und Salzen des Salmeterols, Verfahren zu deren Herstellung sowie deren Verwendung bei der Therapie von Atemwegserkrankungen.

### Hintergrund der Erfindung

Die Verbindung Tiotropiumbromid, ein Salz des Tiotropiums, ist aus der Europäischen Patentanmeldung EP 418 716 A1 bekannt und weist die folgende chemische Struktur auf:

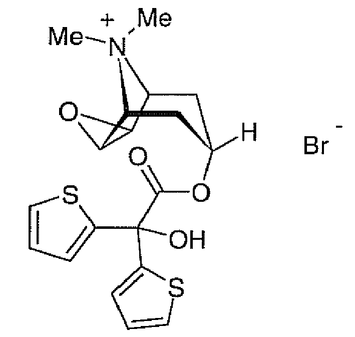

Diese Verbindung kann auch auch durch den chemischen Namen (1α,2β,4β,5α,7β)-7-[(hydroxydi-2-thienylacetyl)oxy]-9,9-dimethyl-3-oxa-9-azoniatricyclo[3.3.1.0^{2,4}] nonane-bromid bezeichnet werden und besitzt wertvolle pharmakologische Eigenschaften. Die Bezeichnung Tiotropium ist im Rahmen der vorliegenden Erfindung als Bezugnahme auf das freie Kation zu verstehen.

Sie, wie auch andere Salze des Tiotropiums, stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumsalzen erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten. Die inhalative Applikation kann ferner mittels geeigneter Lösungen des Tiotropiumsalzes erfolgen.

### Detaillierte Beschreibung der Erfindung

Überraschenderweise wurde gefunden, daß ein unerwartet vorteilhafter therapeutischer Effekt, insbesondere ein synergistischer Effekt bei der Behandlung von entzündlichen oder obstruktiven Atemwegserkrankungen beobachtet wird, wenn ein oder mehrere Tiotropiumsalze (**1**) in Kombination mit ein oder mehreren Salmeteroisalzen (**2**) zur Anwendung gelangen.

Hierdurch lassen sich beispielsweise unerwünschte Nebenwirkungen, die häufig bei der Applikation von β-Mimetika, wie Salmeterol, am Menschen beobachtet werden deutlich verringern. Als zentrale Nebenwirkungen von β-Mimetika seien beispielsweise allgemeine Unruhe, Erregung, Schlaflosigkeit, Angst, Fingerzittern, Schweißausbrüche und Kopfschmerzen genannt.

Die Bezeichnung Tiotropium ist im Rahmen der vorliegenden Erfindung als Bezugnahme auf das freie Kation (**1'**) zu verstehen. Eine Bezugnahme auf Salmeterol ist im Rahmen der vorliegenden Erfindung als Bezugnahme auf die freie Base (**2'**) zu verstehen.

Die erfindungsgemäßen Wirkstoffkombinationen sind überraschenderweise ferner sowohl durch einen raschen Wirkungseintritt, als auch durch eine langandauernde Wirkdauer gekennzeichnet. Dies ist von hoher Bedeutung für das Wohlbefinden des Patienten, da er einerseits nach Applikation der Kombination eine rasche Verbesserung seines Zustands verspürt und andererseits aufgrund der langen Wirkdauer eine einmal pro Tag erfolgende Applikation ausreichend ist.
Die vorstehend genannten Effekte werden sowohl bei gleichzeitiger Applikation innerhalb einer einzigen Wirkstoffformulierung als auch bei sukzessiver Applikation der beiden Wirkstoff in getrennten Formulierungen beobachtet. Erfindungsgemäß bevorzugt ist die gleichzeitige Applikation der beiden Wirkstoffbestandteile in einer einzigen Formulierung.

Ein Aspekt der vorliegenden Erfindung betrifft ein Arzneimittel, welches ein oder mehrere Tiotropiumsalze **(1)** und ein oder mehrere Salmeterolsalze **(2),** gegebenfalls in Form ihrer Solvate oder Hydrate enthält. Hierbei können die Wirkstoffe entweder gemeinsam in einer einzigen Darreichungsform oder in zwei getrennten Darreichungsformen enthalten sein. Erfindungsgemäß bevorzugt sind Arzneimittel, die die Wirkstoffe **1** und **2** in einer einzigen Darreichungsform enthalten.

Ein weiterer Aspekt der vorliegenden Erfindung betrifft ein Arzneimittel, welches neben therapeutisch wirksamen Mengen von **1** und **2** einen pharmazeutisch verträglichen Hilfsstoff enthält. Ein Aspekt der vorliegenden Erfindung betrifft ein Arzneimittel, welches neben therapeutisch wirksamen Mengen von **1** und **2** keinen pharmazeutisch verträglichen Hilfsstoff enthält.

Die vorliegende Erfindung betrifft ferner die Verwendung von **1** und **2** zur Herstellung eines therapeutisch wirksame Mengen von **1** und **2** enthaltenden Arzneimittels zur Behandlung von entzündlichen oder obstruktiven Atemwegserkrankungen, insbesondere von Asthma oder COPD durch simultane oder sukzessive Applikation.

Die vorliegende Erfindung zielt ferner auf die simultane oder sukzessive Verwendung therapeutisch wirksamer Dosen der Kombination vorstehender Arzneimittel **1** und **2** zur Behandlung von entzündlichen oder obstruktiven Atemwegserkrankungen, insbesondere von Asthma oder COPD.

Unter den im Rahmen der vorliegenden Erfindung einsetzbaren Tiotropiumsalzen **1** sind die Verbindungen zu verstehen, die neben Tiotropium als Gegenion (Anion) Chlorid, Bromid, Iodid, Methansulfonat, para-Toluolsulfonat oder Methylsulfat enthalten. Im Rahmen der vorliegenden Erfindung sind von allen Tiotropiumsalzen das Methansulfonat, Chlorid, Bromid oder Iodid bevorzugt, wobei dem Methansulfonat oder dem Bromid besondere Bedeutung zukommt. Von erfindungsgemäß herausragender Bedeutung ist das Tiotropiumbromid.

Unter Salzen des Salmeterols **2** werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure, Xinafonsäure oder Maleinsäure sind, mit der Maßgabe, daß **2** nicht für Salmeterolxinafoat stehen kann, wenn **1** Tiotropiumbromid bedeutet. Gegebenenfalls können zur Herstellung der Salmeterolsalze auch Mischungen der vorgenannten Säuren eingesetzt werden.
Erfindungsgemäß bevorzugt sind die Salmeterolsalze **2** ausgewählt aus der Gruppe bestehend aus Hydrochlorid, Hydrobromid, Sulfat, Phosphat und Methansulfonat. Besonders bevorzugt sind die Salze von **2** ausgewählt aus Hydrochlorid und Sulfat, von denen die Sulfate besonders bevorzugt sind. Erfindungsgemäß von herausragender Bedeutung ist Salmeterol x ½ H₂SO₄.

In den erfindungsgemäßen Wirkstoffkombinationen aus **1** und **2** können die Bestandteile **1** und **2** in Form ihrer Enantiomere, Gemische der Enantiomere oder in Form der Racemate enthalten sein.

Die Verhältnisse, in denen die beiden Wirkstoffe **1** und **2** in die erfindungsgemäßen Wirkstoffkombinationen eingesetzt werden können, sind variabel. Die Wirkstoffe **1** und **2** können gegebenfalls in Form ihrer Solvate oder Hydrate vorliegen. Je nach Wahl der Salze **1** bzw. **2** variieren die im Rahmen der vorliegenden Erfindung einsetzbaren Gewichtsverhältnisse aufgrund des unterschiedlichen Molekulargewichts der verschiedenen Salzformen. Den nachfolgend angegebenen Gewichtsverhältnissen wurden daher das Tiotropiumkation **1**' sowie die freie Base des Salmeterols **2**' zugrunde gelegt. Die erfindungsgemäßen Wirkstoffkombinationen können **1'** und **2'** in Gewichtsverhältnissen enthalten, die in einem Bereich von 1:300 bis 30:1, bevorzugt von 1:230 bis 20:1, besonders bevorzugt von 1:150 bis 10:1, ferner bevorzugt von 1:50 bis 5:1, besonders bevorzugt von 1:35 bis 2:1. Erfindungsgemäß von besonderem Interesse sind Arzneimittel, enthaltend die Kombination aus **1'** und **2'** in einem Gewichtsverhältnis im Bereich von 1:25 bis 1:1. bevorzugt in einem Bereich von 1:10 bis 1:2, besonders bevorzugt in einem Bereich von 1:5 bis 1:2,5.

Beispielsweise und ohne den Umfang der Erfindung darauf zu beschränken, können bevorzugte erfindungsgemäße Kombinationen aus **1** und **2** Tiotropium **1'** und Salmeterol **2'** in den folgenden Gewichtsverhältnissen enthalten: 1:40; 1:20; 1:11,1; 1:10; 1:5,6; 1:5; 1:2,8; 1:2,5; 1:1,4; 1:1,25; 1,44:1, 1,6:1.

Die Anwendung der erfindungsgemäßen Arzneimittel enthaltend die Kombinationen aus **1** und **2** erfolgt üblicherweise so, daß Tiotropium **1'** und Salmeterol **2'** gemeinsam in Dosierungen von 0,01 bis 10000µg, bevorzugt von 0,1 bis 2000µg, besonders bevorzugt von 1 bis 1000µg, ferner bevorzugt von 5 bis 500µg, erfindungsgemäß bevorzugt von 10 bis 200µg, bevorzugt von 20 bis 100µg, höchst bevorzugt von 30 bis 70µg pro Einmalgabe.
Beispielsweise enthalten erfindungsgemäße Kombinationen aus **1** und **2** eine solche Menge an Tiotropium **1'** und Salmeterol **2',** daß die Gesamtdosierung pro Einmalgabe 30µg, 35µg, 45µg, 55µg, 60µg, 65µg, 90µg, 105µg, 110µg, 110µg, 140µg oder ähnliches beträgt. Bei diesen Dosierungsbereichen sind die Wirkstoffe **1'** und 2' in den vorhergehend beschriebenen Gewichtsverhältnissen enthalten.

Beispielsweise und ohne den Umfang der Erfindung darauf zu beschränken, können die erfindungsgemäßen Kombinationen aus **1** und **2** eine solche Menge an Tiotropium **1'** und Salmeterol **2'** enthalten, daß pro Einmalgabe 5µg **1**' und 25µg **2'**, 5µg **1'** und 50µg **2',** 5µg **1'** und 100µg **2',** 5µg **1'** und 200µg **2',** 10µg **1'** und 25µg **2',** 10µg **1'** und 50µg **2',** 10µg **1'** und 100µg **2',** 10µg **1'** und 200µg **2',** 18µg **1'** und 25µg **2',** 18µg **1'** und 50µg **2',** 18µg **1'** und 100µg **2',** 18µg **1'** und 200µg **2',** 20µg **1'** und 25µg **2'**, 20µg **1'** und 50µg **2'**, 20µg **1'** und 100µg **2'**, 20µg **1'** und 200µg **2'**, 36µg **1'** und 25µg **2'**, 36µg **1'** und 50µg **2'**, 36µg **1'** und 100µg **2'**, 36µg **1'** und 200µg **2'**, 40µg **1'** und 25µg **2'**, 40µg **1'** und 50µg **2',** 40µg **1**' und 100µg **2'** oder 40µg **1'** und 200µg **2**' appliziert werden.

Wird als erfindungsgemäß bevorzugte Kombination aus **1** und **2** die Wirkstoffkombination verwendet, in der **1** Tiotropiumbromid bedeutet und in der **2** für Salmeterol x ½H₂SO₄ steht, entsprechen die vorstehend beispielhaft genannten pro Einmalgabe applizierten Wirkstoffmengen von **1'** und **2'** den nachfolgenden pro Einmalgabe applizierten Mengen an **1** und **2**: 6µg **1** und 27,9µg **2**, 6µg **1** und 55,9µg **2,** 6µg **1** und 111,8µg **2**, 6µg **1** und 223,6µg **2**, 12µg **1** und 27,9µg **2**, 12µg **1** und 55,9µg **2**, 12µg **1** und 111,8µg **2**, 12µg **1** und 223,6µg **2**, 21,7µg **1** und 27,9µg **2**, 21,7µg **1** und 55,9µg **2**, 21,7µg **1** und 111,8µg **2**, 21,7µg **1** und 223,6µg **2**, 24,1µg **1** und 27,9µg **2**, 24,1µg **1** und 55,9µg **2**, 24,1µg **1** und 111,8µg **2**, 24,1µg **1** und 223,6µg **2**, 43,3µg **1** und 27,9µg **2**, 43,3µg **1** und 55,9µg **2**, 43,3µg **1** und 111,8µg **2**, 43,3µg **1** und 223,6µg **2**, 48,1µg **1** und 27,9µg **2**, 48,1µg **1** und 55,9µg **2**, 48,1µg **1** und 111,8µg **2** oder 48,1µg **1** und 223,6µg **2.**

Wird in der erfindungsgemäß bevorzugten Kombination aus **1** und **2**, in der **2** für Salmeterol x ½H₂SO₄ steht als **1** beispielsweise das Tiotropiumbromidmonohydrat eingesetzt, entsprechen die vorstehend beispielhaft genannten pro Einmalgabe applizierten Wirkstoffmengen von **1'** und **2**' den nachfolgenden pro Einmalgabe applizierten Mengen an **1** und **2**: 6,2µg **1** und 27,9µg **2**, 6,2µg **1** und 55,9µg **2**, 6,2µg **1** und 111,8µg **2**, 6,2µg **1** und 223,6µg **2**, 12,5µg **1** und 27,9µg **2**, 12,5µg **1** und 55,9µg **2**, 12,5µg **1** und 111,8µg **2**, 12,5µg **1** und 223,6µg **2**, 22,5µg **1** und 27,9µg **2**, 22,5µg **1** und 55,9µg **2**, 22,5µg **1** und 111,8µg **2**, 22,5µg **1** und 223,6µg **2**, 25µg **1** und 27,9µg **2**, 25µg **1** und 55,9µg **2**, 25µg **1** und 111,8µg **2**, 25µg **1** und 223,6µg **2**, 45µg **1** und 27,9µg **2**, 45µg **1** und 55,9µg **2**, 45µg **1** und 111,8µg **2**, 45µg **1** und 223,6µg **2**, 50µg **1** und 27,9µg **2**, 50µg **1** und 55,9µg **2**, 50µg **1** und 111,8µg **2** oder 50µg **1** und 223,6µg **2**.

Die Applikation der erfindungsgemäßen Wirkstoffkombinationen aus **1** und **2** erfolgt bevorzugt auf inhalativem Wege. Hierzu müssen die Bestandteile **1** und **2** in inhalierbaren Darreichungsformen bereitgestellt werden.
Als inhalierbare Darreichungsformen kommen Inhalationspulver, treibgashaltige Dosieraerosole oder treibgasfreie Inhalationslösungen in Betracht. Erfindungsgemäße Inhalationspulver enthaltend die Wirkstoffkombination aus **1** und **2** können allein aus den genannten Wirkstoffen oder aus einem Gemisch der genannten Wirkstoffe mit physiologisch verträglichen Hilfsstoffen bestehen. Im Rahmen der vorliegenden Erfindung sind von dem Begriff treibgasfreie Inhaltionslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

### A) Inhalationspulver enthaltend die erfindungsgemäßen Wirkstoffkombinationen aus 1 und 2:

Die erfindungsgemäßen Inhaltionspulver können **1** und **2** entweder allein oder im Gemisch mit geeigneten physiologisch unbedenkliche Hilfsstoffen enthalten.

Sind die Wirkstoffe **1** und **2** im Gemisch mit physiologisch unbedenklichen Hilfsstoffen enthalten, können zur Darstellung dieser erfindungsgemäßen lnhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1** und **2**, vorzugsweise mit einer mittleren Teilchengröße von 0,5 bis 10µm, besonders bevorzugt von 1 bis 6µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhaltionspulver können entweder in Form einer einzigen Pulvermischung, die sowohl **1** als auch **2** enthält oder in Form von separaten Inhalationspulvern, die lediglich **1** und **2** enthalten bereitgestellt und appliziert werden.

Die erfindungsgemäßen lnhalationspulver können mittels aus dem Stand der Technik bekannten Inhalatoren appliziert werden.
Erfindungsgemäße lnhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2** physiologisch unbedenkliche Hilfsstoff enthalten, allerdings in Kapseln abgefüllt (zu sogenannten lnhaletten), die in Inhalatoren wie beispielsweise in der WO 94/28958 beschrieben, zur Anwendung gelangen.
Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1'** und **2'** genannten Dosierungen pro Einmalgabe.

### B) Treibgashaltige Inhalationsaerosole enthaltend die erfindungsgemäßen Wirkstoffkombinationen aus 1 und 2:

Erfindungsgemäße treibgashaltige Inhaltionsaerosole können **1** und **2** im Treibgas gelöst oder in dispergierter Form enthalten. Hierbei können **1** und **2** in getrennten Darreichungsformen oder in einer gemeinsamen Darreichungsform enthalten sein, wobei **1** und **2** entweder beide gelöst, beide dispergiert oder jeweils nur eine Komponente gelöst und die andere dispergiert enthalten sein können.
Die zur Herstellung der erfindungsgemäßen Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie chlorierten und/oder fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus der Gruppe bestehend aus TG11, TG12, TG134a und TG227. Von den vorstehend genannten halogenierten Kohlenwasserstoffen sind erfindungsgemäß das TG134a (1,1,1,2-Tetrafluorethan) und das TG227 (1,1,1,2,3,3,3-Heptafluorpropan) und Mischungen derselben bevorzugt.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhaltionsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2.**

Liegen die Wirkstoffe **1** und/oder **2** in dispergierter Form vor weisen die Wirkstoffteilchen bevorzugt eine mittlere Teilchengröße von bis zu 10µm, bevorzugt von 0,1 bis 5 µm, besonders bevorzugt von 1 bis 5 µm auf.

Die vorstehend genannten erfindungsgemäßen treibgashaltigen Inhaltionaerosole können mittels im Stand der Technik bekannten Inhalatoren (MDls = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, daß sie vorstehend beschriebene erfindungsgemäße treibgashaltige Aerosole enthalten.
Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaitigen Inhaltionsaerosolen sind aus dem Stand der Technik bekannt.

### C) Treibgasfreie Inhaltionslösungen enthaltend die erfindungsgemäßen Wirkstoffkombinationen aus 1 und 2:

Besonders bevorzugt erfolgt die Applikation der erfindungsgemäßen Wirkstoffkombination in Form von treibgasfreien Inhalationslösungen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Bevorzugtes Lösungsmittel ist Wasser ohne ethanolischen Zusatz. Die **1** und **2** , getrennt oder gemeinsam enthaltenden Lösungen werden mit geeigneten Säuren auf einen pH-Wert von 2 bis 7, bevorzugt von 2 bis 5, besonders bevorzugt von 2,5 bis 3,5 eingestellt. Höchst bevorzugt weisen erfindungsgemäße Inhaltionslösungen, die gemeinsam **1** und **2** enthalten einen pH-Wert von etwa 2,9 auf. Zur Einstellung dieses pH-Werts können Säuren ausgewählt aus anorganischen oder organischen Säuren Verendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff oder im Fall von Kombinationspräparaten mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg / 100 ml, bevorzugt unter 50 mg/100 ml, besonders beovorzugt unter 20 mg/ 100 ml.
Generell sind solche Inhaltionslösungen bevorzugt, in denen der Gehalt an Natriumedetat bei 0 bis 10mg/100ml liegt.

Den erfindungsgemäßen treibgasfreien Inhaltionslösungen können Co-Solventien und/oder weitere Hilfsstoffe zugesetzt werden.
Bevorzugte Co-Solventlen sind solche, die Hydroxylgruppen oder andere polare Gruppen enthalten, beispielsweise Alkohole - insbesondere lsopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu denZusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 20 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (insbesondere Figuren 6a und 6b) ausführlich beschrieben. Die dort beschriebenen Vernebler (Devices) sind auch unter der Bezeichnung Respimat^{®} bekannt.

Dieser Vernebler (Respimat^{®}) kann vorteilhaft zur Erzeugung der erfindungsgemäßen inhalierbaren Aerosole enthaltend die Wirkstoffkombination aus **1** und **2** eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

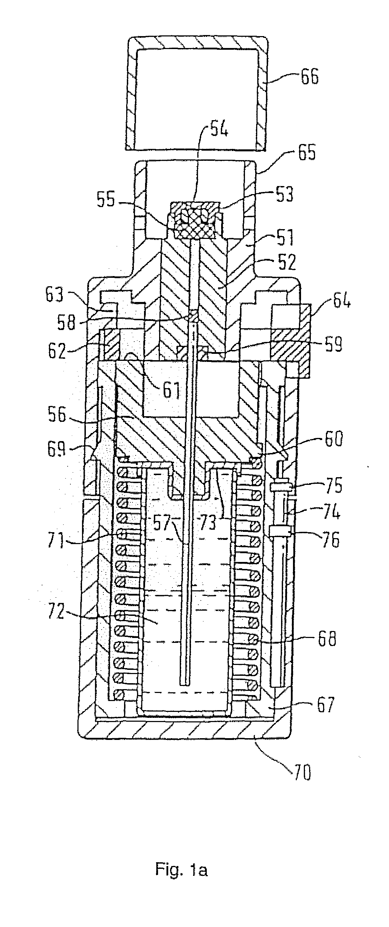

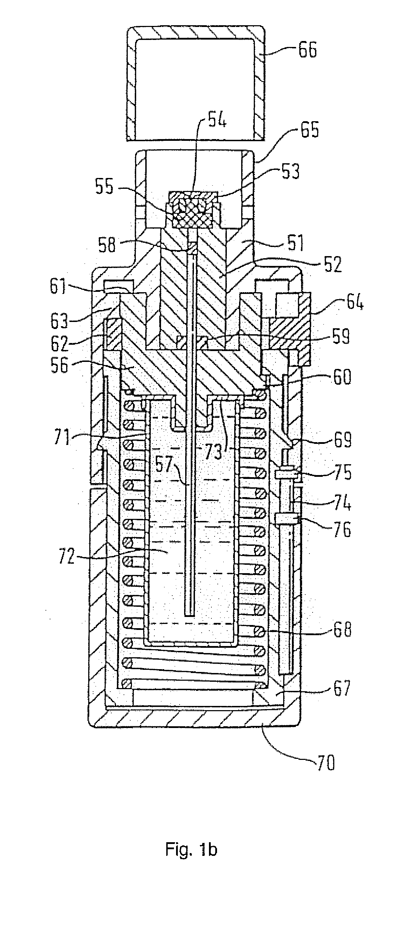

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsfilansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 1a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 1 a zeigt einen Längsschnitt durch den Zerstäuber bei gespannter Feder, Figur 2 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat^{®}) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Dementsprechend betrifft ein weiterer Aspekt der vorliegenden Erfindung Arzneimittel in Form von wie vorstehend beschriebenen treibgasfreien Inhaltionslösungen in Verbindung mit einer zur Verabreichung dieser Lösungen geeigneten Vorrichtung, bevorzugt in Verbindung mit dem Respimat^{®}. Bevorzugt zielt die vorliegende Erfindung auf treibgasfreie Inhaltionslösungen gekennzeichnet durch die erfindungsgemäßen Wirkstoffkombination aus **1** und **2** in Verbindung mit der unter der Bezeichnung Respimat^{®} bekannten Vorrichtung. Ferner betrifft die vorliegende Erfindung vorstehend genannte Vorrichtungen zur Inhalation, bevorzugt den Respimat^{®}, dadurch gekennzeichnet, daß sie vorstehend beschriebene erfindungsgemäße treibgasfreie Inhaltionslösungen enthalten.

Die erfindungsgemäßen treibgasfreien Inhalationslösungen können neben den vorstehend, zur Applikation im Respimat vorgesehenen Lösungen auch als Konzentrate oder sterile gebrauchsfertige lnhalationslösungen vorliegen. Aus den Konzentraten lassen sich beispielsweise durch Zugabe von isotonischen Kochsalzlösungen gebrauchsfertige Inhalationslösungen generieren. Sterile gebrauchsfertige Inhalationslösungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen, die als Konzentrate oder sterile gebrauchsfertige Lösungen vorliegen, in Verbindung mit einer zur Verabreichung dieser Lösungen geeigneten Vorrichtung, dadruch gekennzeichnet, daß s sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Ausgangsmaterialien

### Tiotropiumbromid:

Das in den nachfolgenden Formulierungsbeispielen eingesetzte Tiotropiumbromid kann wie in der Europäischen Patentanmeldung EP 418 716 A1 beschrieben, erhalten werden.

Zur Herstellung der erfindungsgemäßen Inhalationspulver kann ebenfalls kristallines Tiotropiumbromidmonohydrat eingesetzt werden. Dieses kristalline Tiotropiumbromidmonohydrat ist gemäß nachfolgend beschriebener Vorgehensweise erhältlich.
In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromidmonohydrat (86 % d. Th.)

Das so erhaltene kristalline Tiotropiumbromidmonohydrat wird nach bekannten Verfahren mikronisiert, um den Wirkstoff in Form der mittleren Teilchengröße bereitzustellen, die den erfindungsgemäßen Spezifikationen entspricht.

### Salmeterol x ½H₂SO₄:

Wird in den nachfolgenden Ausführungsbeispielen auf Salmeterol x ½ H₂SO₄ Bezug genommen, so wurde dieses wie folgt erhalten:
Eine Suspension aus 2.5 g (4.15 mmol) Salmeterolxinafoat wird in 6 ml Ethanol gelöst. Unter Rühren wird eine Lösung von 0.14 ml 98%iger Schwefelsäure langsam zu der Suspension gegeben. Es wird bis zur vollständigen Lösung auf 35-40 °C erwärmt. Anschließend wird mit 10 ml Diethylether verdünnt und die Lösung mit Salmeteroisulfat angeimpft. Das Salmeterolsulfat wird nach 1.5 Stunden abgesaugt und mit je 20 ml kaltem Ethanol, Aceton und Diethylether gewaschen.
Man erhält 1.5 g (78%) Salmeterol-½-sulfat

### Formulierungsbeispiele

### A) Inhaltionspulver:

### 1)

| **Bestandteile** | **µg pro Kapsel** |
|---|---|
| Tiotropiumbromid | 10,8 |
| Salmeterol x ½ H₂SO₄ | 27,9 |
| Lactose | 4961,3 |
| **Summe** | 5000 |

### 2)

| **Bestandteile** | **µg pro Kapsel** |
|---|---|
| Tiotropiumbromid | 21,7 |
| Salmeterol x ½ H₂SO₄ | 55,9 |
| Lactose | 4922,4 |
| **Summe** | 5000 |

### 3)

| **Bestandteile** | **µg pro Kapsel** |
|---|---|
| Tiotropiumbromid x H₂O | 22,5 |
| Salmeterol x ½ H₂SO₄ | 55,9 |
| Lactose | 4921,6 |
| **Summe** | 5000 |

### B) Treibrgashaltige Inhaltionsaerosole:

### 1) Suspensionsaerosol:

| **Bestandteile** | **Gew-%** |
|---|---|
| Tiotropiumbromid | 0,015 |
| Salmeterol x ½ H₂SO₄ | 0,066 |
| Sojalecithin | 0,2 |
| TG11 : TG12 = 2:3 | ad 100 |

### 2) Suspensionsaerosol:

| **Bestandteile** | **Gew-%** |
|---|---|
| Tiotropiumbromid | 0,029 |
| Salmeterol x ½ H₂SO₄ | 0,033 |
| Ethanol, absolut | 0,5 |
| Isopropylmyristat | 0,1 |
| TG 227 | ad 100 |

### 3) Lösungsaerosol:

| **Bestandteile** | **Gew-%** |
|---|---|
| Tiotropiumbromid | 0,042 |
| Salmeterol x ½ H₂SO₄ | 0,047 |
| Ethanol, absolut | 30 |
| Wasser, gereinigt | 1,5 |
| Zitronensäure, wasserfrei | 0,002 |
| TG 134a | ad 100 |

### C) Treibgasfreie Inhaltionslösungen:

### 1) Lösung zur Anwendung im Respimat^{®}:

| **Bestandteile** | **mg/100mL** |
|---|---|
| Tiotropiumbromid | 148,5 |
| Salmeterol x ½ H₂SO₄ | 276,7 |
| Benzalkoniumchlorid | 10 |
| Natriumedetat | 10 |
| Salzsäure (aq) | ad pH 2,9 |
| Wasser | ad 100mL |

Verwendung der Lösung im Respimat führt zu einer Dosierung von 10µg pro Dosierung **1** und 25µg/Dosierung **2.**

### 2) Lösung zur Anwendung im Respimat^{®}:

| **Bestandteile** | **mg/100mL** |
|---|---|
| Tiotropiumbromid | 148,5 |
| Salmeterol x ½ H₂SO₄ | 276,7 |
| Benzalkoniumchlorid | 10 |
| Salzsäure (aq) | ad pH 2,9 |
| Wasser | ad 100mL |

Verwendung der Lösung im Respimat führt zu einer Dosierung von 10µg pro Dosierung **1** und 25µg/Dosierung **2**.

### 3) Lösung zur Anwendung im Respimat^{®}:

| **Bestandteile** | **mg/100mL** |
|---|---|
| Tiotropiumbromid | 297,1 |
| Salmeterol x ½ H₂SO₄ | 276,7 |
| Benzalkoniumchlorid | 10 |
| Natriumedetat | 10 |
| Salzsäure (aq) | ad pH 2,9 |
| Wasser | ad 100mL |

Verwendung der Lösung im Respimat führt zu einer Dosierung von 20µg pro Dosierung **1** und 25µg/Dosierung **2**.

### 4) Lösung zur Anwendung im Respimat^{®}:

| **Bestandteile** | **mg/100mL** |
|---|---|
| Tiotropiumbromid | 297,1 |
| Salmeterol x ½ H₂SO₄ | 276,7 |
| Benzalkoniumchlorid | 10 |
| Salzsäure (aq) | ad pH 2,9 |
| Wasser | ad 100mL |

Verwendung der Lösung im Respimat führt zu einer Dosierung von 20µg pro Dosierung **1** und 25µg/Dosierung **2**.

### 5) Lösung zur Anwendung im Respimat^{®}:

| **Bestandteile** | **mg/100mL** |
|---|---|
| Tiotropiumbromid | 148,5 |
| Salmeterol x ½ H₂SO₄ | 1106,3 |
| Benzalkoniumchlorid | 8 |
| Natriumedetat | 50 |
| Salzsäure (aq) | ad pH 2,5 |
| Wasser | ad 100mL |

Verwendung der Lösung im Respimat führt zu einer Dosierung von 10µg pro Dosierung **1** und 100µg/Dosierung **2**.

### 6) Lösung zur Anwendung im Respimat®:

| **Bestandteile** | **mg/100mL** |
|---|---|
| Tiotropiumbromid | 1,5 |
| Salmeterol x ½ H₂SO₄ | 276,7 |
| Benzalkoniumchlorid | 8 |
| Natriumedetat | 10 |
| Salzsäure (aq) | ad pH 2,5 |
| Wasser | ad 100mL |

Verwendung der Lösung im Respimat führt zu einer Dosierung von 0,1µg pro Dosierung **1** und 25µg/Dosierung **2**.

### 7) Lösung zur Anwendung im Respimat^{®}:

| **Bestandteile** | **mg/100mL** |
|---|---|
| Tiotropiumbromid | 14,9 |
| Salmeterol x ½ H₂SO₄ | 1106,32 |
| Benzalkoniumchlorid | 10 |
| Natriumedetat | 50 |
| Salzsäure (aq) | ad pH 3,5 |
| Wasser | ad 100mL |

Verwendung der Lösung im Respimat führt zu einer Dosierung von 1µg pro Dosierung **1** und 100µg/Dosierung **2.**

### 8) Lösung zur Anwendung im Respimat^{®}:

| **Bestandteile** | **mg/100mL** |
|---|---|
| Tiotropiumbromid | 1486,1 |
| Salmeterol x ½ H₂SO₄ | 1106,32 |
| Benzalkoniumchlorid | 10 |
| Natriumedetat | 10 |
| Salzsäure (aq) | ad pH 3,5 |
| Wasser | ad 100mL |

Verwendung der Lösung im Respimat führt zu einer Dosierung von 100µg pro Dosierung **1** und 100µg/Dosierung **2.**

### 9) Konzentrierte Lösung:

| **Bestandteile** | **mg/100mL** |
|---|---|
| Tiotropiumbromid | 1486,1 |
| Salmeterol x ½ H₂SO₄ | 11063,2 |
| Benzalkoniumchlorid | 20 |
| Natriumedetat | 100 |
| Salzsäure (aq) | ad pH 3,5 |
| Wasser | ad 100mL |

## Patentansprüche

1. Arzneimittel **gekennzeichnet durch** einen Gehalt an einem oder mehreren Tiotropiumsalzen (**1**) in Kombination mit einem oder mehreren Salmeterolsalzen (**2**), gegebenenfalls in Form ihrer Enantiomere, Gemische der Enantiomere oder in Form der Racemate, gegebenenfalls in Form der Solvate oder Hydrate sowie gegebenenfalls gemeinsam mit einem pharmazeutisch verträglichen Hilfsstoff.

2. Arzneimittel nach Anspruch 1, **dadurch gekennzeichnet, daß** die Wirkstoffe **1** und **2** entweder gemeinsam in einer einzigen Darreichungsform oder in zwei getrennten Darreichungsformen enthalten sind.

3. Arzneimittel nach einem der Ansprüche 1 oder 2, **dadurch gekennzeichnet, daß 1** in Form des Chlorids, Bromids, Iodids, Methansulfonats, para-Toluolsulfonats oder Methylsulfats, bevorzugt in Form des Bromids enthalten ist.

4. Arzneimittel nach einem der Ansprüche 1 bis 3, **dadurch gekennzeichnet, daß** 2 ausgewählt ist aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Xinafonsäure oder Maleinsäure, mit der Maßgabe, daß **2** nicht für Salmeterolxinafoat stehen kann, wenn **1** Tiotropiumbromid bedeutet.

5. Arzneimittel nach Anspruch 4, **dadurch gekennzeichnet, daß 2** ausgewählt ist aus den Salzen Hydrochlorid, Hydrobromid, Sulfat, Phosphat und Methansulfonat, bevorzugt aus Hydrochlorid und Sulfat.

6. Arneimittel nach einem der Ansprüche 1 bis 5, **dadurch gekennzeichnet, daß** die Gewichtsverhältnisse von Tiotropium **1'** zu Salmeterol **2'** ,in einem Bereich von 1:300 bis 30:1, bevorzugt von 1:230 bis 20:1 liegen.

7. Arzneimittel nach einem der Ansprüche 1 bis 6, **dadurch gekennzeichnet, daß** eine einmaliger Applikation einer Dosierung der Wirkstoffkombination **1'** und **2**' von 0,01 bis 1000µg, bevorzugt von 0,1 bis 200µg entspricht.

8. Arzneimittel nach einem der Ansprüche 1 bis 7, **dadurch gekennzeichnet, daß** es in Form einer für die Inhalation geeigneten Darreichungsform vorliegt.

9. Arzneimittel nach Anspruch 8, **dadurch gekennzeichnet, daß** es sich um eine Darreichungsform ausgewählt aus der Gruppe Inhalationspulver, treibgashaltige Dosieraerosole und treibgasfreie Inhalationslösungen handelt.

10. Arzneimittel nach Anspruch 9, **dadurch gekennzeichnet, daß** es ein Inhalationspulver ist, welches **1** und **2** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hilfsstoffe miteinandenthält.

11. Inhalationspulver nach Anspruch 10, **dadurch gekennzeichnet, daß** der Hilfsstoff eine maximale mittlere Teilchengröße von bis zu 250µm, bevorzugt zwischen 10 und 150µm aufweist.

12. Kapseln **gekennzeichnet durch** einen Gehalt an Inhaltionspulver nach Anspruch 10 oder 11.

13. Arzneimittel nach Anspruch 9, **dadurch gekennzeichnet, daß** es ein Inhalationspulver ist, welches als Bestandteile lediglich die Wirkstoffe **1** und **2** enthält.

14. Arzneimittel nach Anspruch 9, **dadurch gekennzeichnet, daß** es sich um ein treibgashaltiges Inhalationsaerosol handelt, welches **1** und **2** in gelöster oder dispergierter Form enthält.

15. Treibgashaltiges Inhalationsaerosol nach Anspruch 14, **dadurch gekennzeichnet, daß** es als Treibgas Kohlenwasserstoffe wie n-Propan, n-Butan oder Isobutan oder Halogenkohlenwasserstoffe wie chlorierte und/oder fluorierte Derivate des Methans, Ethans, Propans, Butans, Cyclopropans oder Cyclobutans enthält.

16. Treibgashaltiges Inhaltionsaerosol nach Anspruch 15, **dadurch gekennzeichnet, daß** das Treibgas TG11, TG12, TG134a, TG227 oder Gemische davon, bevorzugt TG134a, TG227 oder ein Gemisch davon darstellt.

17. Treibgashaltiges Inhalationsaerosol nach Anspruch 14, 15 oder 16, dadruch **gekennzeichnet**, daß es gegebenenfalls einen oder mehrere weitere Bestandteile ausgewählt aus der Gruppe bestehend aus Kosolventien, Stabilisatoren, oberflächenaktive Mittel (surfactants), Antioxidantien, Schmiermittel und Mittel zur Einstellung des pH-Werts enthält.

18. Treibgashaltiges Inhalationsaerosol nach einem der Ansprüche 14 bis 17, **dadurch gekennzeichnet, daß** es bis zu 5 Gew-% an Wirkstoff **1**' und/oder **2'** enthalten kann.

19. Arzneimittel nach Anspruch 9, **dadurch gekennzeichnet, daß** es sich um eine treibgasfreie Inhalationslösung handelt, die als Lösemittel Wasser, Ethanol oder ein Gemisch aus Wasser und Ethanol enthält.

20. Inhaltionslösung nach Anspruch 19, **dadurch gekennzeichnet, daß** der pH der Lösung 2 - 7, bevorzugt 2 - 5 beträgt.

21. Inhaltionslösung nach Anspruch 20, **dadurch gekennzeichnet, daß** der pH mittels einer Säure ausgewählt aus der Gruppe bestehend aus Salzsäure, Bromwasserstoffsäure, Salpetersäure, Schwefelsäure, Ascorbinsäure, Zitronensäure, Äpfelsäure, Weinsäure, Maleinsäure, Bernsteinsäure, Fumarsäure, Essigsäure, Ameisensäure und Propionsäure oder Gemischen davon, eingestellt wird.

22. Inhalationslösung nach einem der Ansprüche 19 bis 21, **dadurch gekennzeichnet, daß** sie gegebenenfalls weitere Co-Solventien und/oder Hilfsstoffe enthalten.

23. Inhalationslösung nach Anspruch 22, **dadurch gekennzeichnet, daß** sie als Co-Solventien Bestandteile enthalten, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

24. lnhalationslösung nach einem der Ansprüche 22 oder 23, **dadurch gekennzeichnet, daß** sie als Hilfsstoffe oberflächenaktive Stoffe Stabilisatoren, Komplexbildner, Antioxidantien und/oder Konservierungsstoffe, Geschmackstoffe, pharmakologisch unbedenkliche Salze und/oder Vitamine enthalten.

25. Inhalationslösungen nach Anspruch 24, **dadurch gekennzeichnet, daß** sie als Komplexbildner Editinsäure oder ein Salz der Editinsäure, bevorzugt Natriumedetat, enthalten.

26. Inhalationslösungen nach Anspruch 24 oder 25, **dadurch gekennzeichent,** daß sie als Antioxidantien,Verbindungen ausgewählt aus der Gruppe bestehend aus Ascorbinsäure, Vitamin A, Vitamin E und Tocopherole enthalten.

27. Inhalationslösungen nach Anspruch 24, 25 oder 26, **dadurch gekennzeichnet, daß** sie als Konservierungsmittel Verbindungen ausgewählt aus Cetylpyridiniumchlorid, Benzalkoniumchlorid, Benzoesäure und Benzoaten enthalten.

28. Inhalationslösungen nach einem der Ansprüche 22 bis 27, **dadurch gekennzeichnet, daß** sie neben den Wirkstoffen **1** und **2** und dem Lösemittel nur noch Bezalkoniumchlorid und Natriumedetat enthalten.

29. Inhalationslösungen nach einem der Ansprüche 22 bis 27, **dadurch gekennzeichnet, daß** sie neben den Wirkstoffen **1** und **2** und dem Lösemittel nur noch Benzalkoniumchlorid enthalten.

30. Inhalationslösungen nach einem der Ansprüche 19 bis 29, **dadurch gekennzeichnet, daß** es sich um Konzentrate oder sterile gebrauchsfertige Inhalationslösungen handelt.

31. Verwendung einer Inhalationslösung gemäß Anspruch 30 zur Vernebelung in einem energiebetriebenen Stand- oder transportablen Vernebler, der inhalierbare Aerosole mittels Ultraschall oder Druckluft nach dem Venturiprinzip oder anderen Prinzipien erzeugt.

32. Verwendung einer Zusammensetzung nach einem der Ansprüche 1 bis 30 zur Herstellung eines Medikaments zur Behandlung von Atemwegserkrankungen.

33. Verwendung nach Anspruch 33 zur Herstellung eines Medikaments zur Behandlung von Asthma oder COPD.
